# EUROPÄISCHE PATENTSCHRIFT

(11) **EP 1 139 985 B1**
(45) Veröffentlichungstag und Bekanntmachung des Hinweises auf die Patenterteilung: **12.04.2006**
(21) Anmeldenummer: 99962185.7
(22) Anmeldetag: 30.11.1999
(51) Int. Cl.: A61K 8/73, A61Q 1/02, A61Q 19/00

(54) **ALPHA-POLYGLUCANE ENTHALTENDE ZUBEREITUNGEN FÜR DIE TOPISCHE ANWENDUNG**
PREPARATIONS CONTAINING ALPHA POLYGLUCANS FOR TOPICAL APPLICATION
PREPARATIONS CONTENANT DES ALPHA-POLYGLUCANES POUR L'APPLICATION LOCALE

(30) Priorität: 28.12.1998 DE 19860366
(43) Veröffentlichungstag der Anmeldung: 10.10.2001
(73) Patentinhaber: Celanese Ventures GmbH, 65926 Frankfurt am Main (DE)
(72) Erfinder: BENGS, Holger, D-60598 Frankfurt (DE); BRUNNER, Anette, D-91154 Roth (DE); GRANDE, Jürgen, D-65812 Bad Soden (DE); SCHUTH, Silke, D-56412 Ruppach-Goldhausen (DE); BÖHM, Gitte, D-60439 Frankfurt (DE); BRAUNAGEL, Alfred, D-55128 Mainz (DE)
(74) Vertreter: Luderschmidt, Schüler & Partner GbR
(86) Internationale Anmeldenummer: PCT/EP1999/009289
(87) Internationale Veröffentlichungsnummer: WO 2000/038627

(56) Entgegenhaltungen:
- EP-A- 0 487 000
- EP-A- 0 966 949
- WO-A-95/31553
- WO-A-99/11695
- WO-A-99/67412
- US-A- 4 818 751

## Beschreibung

Die vorliegende Erfindung betrifft eine kosmetische oder medizinische Zubereitung für die topische Anwendung enthaltend mindestens ein biotechnisch erhaltenes wasserunlösliches lineares Poly-α-glucan, insbesondere ein Hautpflegemittel.

Die Verwendung von Polysacchariden auf Stärkebasis wie Polyglucanen für kosmetische Zwecke ist seit altersher bekannt.

In der letzten Zeit wurden zunehmend Polysaccharidprodukte für kosmetische und therapeutische Zwecke entwickelt, die spezielle Eigenschaftsprofile aufweisen.
So wird von H. Eggensperger, M. Wilker in SÖFW-Joumal, 123. Jahrgang 8/97, seiten 542 bis 546, "Multiaktivwirksame Polysaccharide, Teil l- Pilzextrakte" für beta-Polyglucane aus Pilzen wie Hefen und deren carboxymethylierte Derivate ein hohe pflegende Wirkung für gereizte, trockene Haut beschrieben.
Besonders vorteilhafte Effekte auf die Haut konnten für beta-1,3-Polyglucane mit beta-1,6- Verknüpfungen nachgewiesen werden, für die zudem eine immunstimulierende Wirkung und Tumoraktivität beobachtet wurde (o.a. Ort, F. Zülli et al., am o.a. Ort, Seiten 535 bis 541).

Auch Cyclodextrine, cyclische alpha-, beta- oder gamma 1,4-Oligosaccharide mit 6 bis 8 Glucaneinheiten, finden steigend Anwendung für die funktionelle Hautpflege, da sie mit einer Vielzahl von Wirkstoffen und pflegenden Substanzen Einschlußverbindungen bilden und dadurch eine retardierte Freisetzung dieser Substanzen am Anwendungsort bewirken können (U. Citernesi, M. Scaiacchitano in Cosmetics and Toiletries magazine, Band 110, März 1995, Seiten 53 bis 61 "Cyclodextrines in functional Dermocosmetics").
Weiter wird vorgeschlagen, biotechnisch oder aus marinen Mollusken gewonnenes Glykogen, ein hochverzweigtes Poly-1,4-α-glucan mit Verzweigungen in 6-Position, für kosmetische Zwecke einzusetzen (M. Pauly, G. Pauly "New Polysaccharides Interest in Care Cosmetology" IN-COSMETICS 1997, Conference Proceedings, Seiten 417-444, Verlag für chemische Industrie, H.Ziolkowsky GmbH,1998).

In der EP-B-0 487 000 wird eine emulsionsförmige kosmetische Zusammensetzung mit 15 bis 40 Gew.% einer enzymatisch entzweigten Stärke vorgeschlagen, wobei die enzymatisch abgebaute Stärke ein lineares Poly-1,4-α-glucan mit 15 bis 65 Anhydroglucoseeinheiten ist.

Obwohl eine Vielzahl von Produkten für die unterschiedlichsten kosmetischen und medizinischen Verwendungszwecke bekannt sind, besteht ein ständiger Bedarf nach neuen verbesserten Produkten. Wünschenswert für die Herstellung hochwertiger Produkte ist insbesondere die einfache Verfügbarkeit von Grundstoffen mit gleichbleibend hoher Qualität und reproduzierbar homogenen Eigenschaftsprofil.

Derartige Grundstoffe können vorteilhaft für die Herstellung von topischen Zubereitungen für empfindliche Haut oder für medizinische Zwecke eingesetzt werden.

Erfindungsgemäß wird eine Zubereitung für die topischen Anwendung zur Verfügung gestellt, nachfolgend auch topische Zubereitung genannt, die als wesentlichen Bestandteil mindestens ein biotechnisch erzeugtes, lineares wasserunlösliches Poly-α-glucan enthält, wie in Anspruch 1 definiert.

Wesentlich ist der erfindungsgemäße Einsatz von mindestens einem wasserunlöslichen linearen Poly-α-1,4,-D-glucan, das biotechnisch erhalten worden ist.

Derartige Poly-α-glucane zeichnen sich durch eine hohe Gleichförmigkeit, Reinheit und einfache Reproduzierbarkeit bei gleichbleibenden Eigenschaften aus. Dies ist insbesondere von Bedeutung für hochwertige Kosmetika und medizinische Zubereitungen, für die eine gleichbleibend hohe Qualität nicht nur erwünscht sondem notwendig ist.
Unter Zubereitungen für die topische Anwendung werden im Sinne der Erfindung ganz allgemein kosmetische Mittel also Körperpflegemittel und dekorative Kosmetika verstanden. Erfindungsgemäß umfaßt der Begriff auch human- und tiermedizinische Zubereitungen, die äußerlich appliziert werden.

Erfindungsgemäße Zubereitungen können Cremes, Kompaktcremes, Lotionen, Masken, Puder in beliebiger Form z.B. flüssig, lose, kompakt, partikelförmig etc., Salben, Salbengrundlagen, Seifen usw. für die dekorative, pflegende oder medizinische Anwendung sein.
Beispiele für die dekorative Kosmetik sind Cremes, Puder, oder Fonds für Make-up, z.B. Rouge, Lidschatten, Lippenstifte.

Im Rahmen der Erfindung bedeutet "biotechnische Herstellung" die Anwendung von biokatalytischen, auch biotransformatorischen, oder fermentativen Prozessen.

Wasserunlösliche lineare Poly-α-glucane hergestellt durch Biokatalyse (auch: Biotransformation) im Rahmen dieser Erfindung bedeutet, daß das lineare Polyglucan durch katalytische Reaktion von monomeren Grundbausteinen wie oligomeren Sacchariden, z.B. von Mono- und/oder Disacchariden, hergestellt wird, indem ein sogenannter Biokatalysator, üblicherweise ein Enzym, unter geeigneten Bedingungen verwendet wird. Man spricht in diesem Zusammenhang auch von "in vitro Biokatalyse".

Wasserunlösliche linerare Polyglucane aus Fermentationen sind im Sprachgebrauch der Erfindung lineare Polyglucane, die durch fermentative Prozesse unter der Verwendung in der Natur vorkommende Organismen, wie Pilzen, Algen, Bazillen, Bakterien oder Protisten oder unter der Verwendung von in der Natur nicht vorkommender Organismen, aber unter Zuhilfenahme von gentechnischen Methoden allgemeiner Definition modifizierten natürlichen Organismen, wie Pilzen, Algen, Bazillen, Bakterien oder Protisten gewonnen werden oder unter Einschaltung und Mithilfe von fermentativen Prozessen gewonnen werden können. Man spricht in diesem Zusammenhang auch von "in vivo Biokatalyse".
Beispiele für derartige Mikroorganismen sind Piichia pastoris, Trichoderma Reseii, Straphylokkus Camosus, Escherichia coli oder Aspergillus Niger.

Vorteilhafte Verfahren für die biotechnische Gewinnung sind z. B. in der WO 95/31553 oder der nicht vorveröffentlichten Patentanmeldung WO 99/67412 der Anmelderin.

Gemäß den dort beschriebenen Verfahren wird eine Saccharoselösung mit Amylosucrase versetzt, wobei unter Spaltung der Zuckerbindung direkt Poly-1,4-α-D-glucan und Fructose gebildet werden.
Weiter geeignete Enzyme sind Polysaccharidsynthasen, Stärkesynthasen, Glycoltransferasen, 1,4-α-D-Glucantransferasen, Glycogensynthasen oder auch Phosphorylasen.

Im Gegensatz zu Poly-α-glucanen, die aus natürlichen Quellen, wie Pflanzen, isoliert werden, weisen die hierbei erhaltenen linearen wasserunlöslichen Polyglucane ein besonders homogenes Eigenschaftsprofil auf, z. B. in bezug auf die Molekulargewichtsverteilung, sie enthalten keine oder allenfalls nur in sehr geringen Mengen unerwünschter Nebenprodukte, die aufwendig abgetrennt werden müssen oder allergene Reaktionen auslösen könnten, und lassen sich exakt spezifiziert auf einfache Weise reproduzieren.
So können bei Bedarf Poly-α-glucane mit unterschiedlichen Eigenschaften wie Molekulargewichten etc. in definierter Weise und einfach reproduzierbar erhalten werden.

Zwar können auch mit der chemischen oder enzymatischen Entzweigung vergleichsweise homogene Produkte erhalten werden. Jedoch verbleibt in vielen Fällen ein Rest an nicht oder nur unzureichend entzweigten Ausgangsmaterial, das nur schwer abgetrennt werden kann.

Wasserunlösliche lineare Poly-α-glucane im Sinne der vorliegenden Erfindung sind . Polysaccharide, die aus Glucanen als monomeren Bausteinen derart aufgebaut sind, daß die einzelnen Bausteine stets in der gleichen Art miteinander verknüpft sind. Jede so definierte Grundeinheit oder Baustein hat genau zwei Verknüpfungen, jeweils eine zu einem anderen Monomer. Davon ausgenommen sind lediglich die beiden Grundeinheiten, die den Anfang bzw. das Ende des Polysaccharids bilden.

Diese haben nur eine Verknüpfung zu einem weiteren Monomer und bilden die Endgruppen des linearen Polyglucans.

Besitzt die Grundeinheit drei oder mehr Verknüpfungen, wird von Verzweigung gesprochen. Dabei ergibt sich aus der Anzahl der Hydroxylgruppen pro 100 Grundeinheiten, die nicht am Aufbau des linearen Polymerrückgrats beteiligt sind und die Verzweigungen ausbilden, der sogenannte Verzweigungsgrad.

Für die Erfindung sind Poly-α-glucane geeignet, die keine Verzweigungen aufweisen, bzw. deren Verzweigungsgrad so minimal ist, daß er mit herkömmlichen Methoden nicht mehr nachweisbar ist

Für die vorliegende Erfindung beziehen sich die Präfixe "alpha" oder "D" allein auf die Verknüpfungen, die das Polymerrückgrat ausbilden und nicht auf die Verzweigungen.

Biotechnische und insbesondere biokatalytische Methoden haben den Vorteil, daß der Verzweigungsgrad kontrollierbar eingestellt werden kann und insbesondere direkt wasserunlösliche lineare Poly-α-glucane erhalten werden können, wie z. B. die Poly-1,4-α-D-glucane, die keine Verzweigungen enthalten, bzw deren Verzweigungsgrad unterhalb der Nachweisgrenze herkömmlicher analytischer Methoden liegt.

Unter dem Begriff "wasserunlösliches Poly-α-glucan" werden für die vorliegende Erfindung Verbindungen verstanden, die nach der Definition des Deutschen Arzneimittelbuches (DAB = Deutsches Arzneimittelbuch, Wissenschaftliche Verlagsgesellschaft mbH, Stuttgart, Govi-Verlag, Frankfurt, Auflage, 1987) entsprechend den Klassen 4 bis 7 unter die Kategorien "wenig lösliche", "schwer lösliche", "sehr schwer lösliche" bzw. "praktisch unlösliche" Verbindungen fallen.

Im Fall der erfindungsgemäß verwendeten Polyglucane bedeutet dies, daß mindestens 98 % der eingesetzten Menge, insbesondere mindestens 99,5 %, unter Normalbedingungen (T = 25 °C +/- 20 %, p= 101325 Pascal +/- 20 %) in Wasser unlöslich ist (entsprechend den Klassen 4 bzw. 5).

Für die vorliegende Erfindung sind schwer lösliche bis praktisch unlösliche Verbindungen, insbesondere sehr schwer lösliche bis praktisch unlösliche Verbindungen, bevorzugt.

"Sehr schwer löslich" entsprechend Klasse 6 kann durch folgende Versuchsbeschreibung veranschaulicht werden:
Ein Gramm des zu untersuchenden Polyglucans werden in 1 l entionisierten Wasser auf 130° C unter einem Druck von 1 bar erhitzt. Die entstehende Lösung bleibt nur kurzzeitig über wenige Minuten stabil. Beim Erkalten unter Normalbedingungen fällt die Substanz wieder aus. Nach Abkühlung auf Raumtemperatur und Abtrennung mittels Zentrifugation können unter Berücksichtigung der experimentellen Verluste mindestens 66 % der eingesetzten Menge zurückgewonnen werden.

Für die vorliegende Erfindung kann das biotechnisch erhaltene Poly-α-glucan als solches eingesetzt werden. Falls erwünscht, kann es einer zusätzlichen Behandlung unterzogen werden.

So können die wasserunlöslichen linearen Poly-α-glucane modifiziert werden, z.B. indem die Poly-α-glucane durch Veresterung und/oder Veretherung in einer oder mehreren nicht an der linearen Verknüpfung beteiligten Positionen chemisch modifiziert werden. Im Fall der bevorzugten 1,4 verknüpften Poly-α-glucane kann die Modifizierung in 2-, 3- und/oder 6-Position erfolgen.

Modifikation im Sinne der Erfindung bedeutet, daß die vorhandenen Hydroxylgruppen, die nicht an der Verknüpfung beteiligt sind, chemisch verändert werden. Dies schließt eine Ringöffnung der Glucaneinheiten aus wie sie z.B. bei der oxidativen Carboxylierung oder der Hydrolyse erfolgt. Maßnahmen für derartige Modifizierungen sind dem Fachmann hinlänglich bekannt.

Die Poly-α-glucane können in Form sogenannter alpha-amylaseresistenter Poly-α-glucane eingesetzt werden wie sie am Beispiel von Poly(1,4-α-D-glucan) in der nicht vorveröffentlichten Patentanmeldung WO 00/02926 der Anmelderin beschrieben sind.

Alpha-amylaseresistente Poly-α-glucane können durch Herstellung einer Suspension oder Dispersion aus wasserunlöslichen Polyglucanen und Wasser, Erwärmen der Suspension oder Dispersion auf eine Temperatur im Bereich von 50 bis 100 °C, Abkühlenlassen der erhaltenen kleisterartigen Mischung auf eine Temperatur im Bereich von 50 °C bis an den Gefrierpunkt, vorzugsweise 35 bis 15 °C, 27 bis 22 °C, 16 bis 0 °C oder 6 bis 2°C, über einen Zeitraum von 1 bis 72 h, vorzugsweise 1 bis 36 h und insbesondere 15 bis 30 h und Retrogradation der kleisterartigen Mischung bei einer gegenüber der Temperatur der erwärmten kleisterartigen Mischung erniedrigten Temperatur in einem Temperaturbereich von 90 bis 4 °C sowie gegebenenfalls Trocknung oder Entwässerung des erhaltenen Produktes erhaltenen werden.

Das Poly-α-glucan kann auch als thermoplastisches Polyglucan eingesetzt werden, das erhältlich ist durch Aufschmelzen von linearem wasserunlöslichen Polyglucan und Hinzufügen von mindestens 20 Gew.%, vorzugsweise mindestens 30 Gew.%, eines Weichmachers wie Sorbitol, Glycerin, deren Kondensationsprodukte und Oligomere, DMSO, Bernsteinsäure, Citronensäure-Monohydrat, Apfelsäure, Weinsäure etc. bei ca. 170 °C.
Eine Beschreibung von geeigneten Maßnahmen und Eigenschaften von thermoplastischen Polyglucanen am Beispiel des linearen wasserunlöslichen Poly-1,4-α-D-glucans gibt die nicht vorveröffentlichte Patentanmeldung WO 00/29477, auf die hierfür ausdrücklich bezug genommen wird.
Zur besseren Einmischbarkeit kann das thermoplastische Poly-α-glucan vorab granuliert werden.

Die Molekulargewichte M_{w} (Gewichtsmittel, bestimmt mittels Gelpermeationschromatographie im Vergleich zu einer Eichung mit. Pullulanstandard) der erfindungsgemäß verwendeten wasserunlöslichen linearen Poly-α-glucane können in einem weiten Bereich von 10³ g/mol bis 10⁷ g/mol variieren. Bevorzugt liegt das Molekulargewicht M_{w} in einem Bereich von 10³ g/mol bis 10⁶ g/mol und besonders bevorzugt von 10³ g/mol bis 10⁵ g/mol. Ein weiterer vorteilhafter Bereich ist von 2 x 10³ bis 8 x 10³. Entsprechende Bereiche gelten für das bevorzugt eingesetzte Poly-1,4-α-D-glucan.
Die Molekulargewichtsverteilung bzw. Polydispersität M_{w}/Mₙ kann ebenfalls in weiten Bereichen je nach Herstellungsverfahren des Polyglucans variieren. Bevorzugte Werte sind von 1,01 bis 50, insbesondere von 1,01 bis 15, wobei kleine Werte besonders bevorzugt sind, z.B. von 1,01 bis 2,5.

Zur Herstellung der topischen Zubereitung kann ein einziges wasserunlösliches lineares Poly-α-glucan oder eine Mischung aus zwei oder mehreren davon eingesetzt werden.
Es können auch Mischungen aus biotechnisch erhaltenen, wasserunlöslichen linearen Poly-α-glucan und Polyglucanen aus anderen Quellen eingesetzt werden.

Aufgrund ihrer Naturidentität kann für die erfindungsgemäß eingesetzten wasserunlöslichen linearen Poly-α-glucane eine ausgezeichnete Biokompatibilität erwartet werden.

Je nach erwünschter Anwendung können die erfindungsgemäßen topischen Zubereitungen geeignete weitere für den jeweiligen Zweck übliche Inhaltsstoffe enthalten.

Beispiele für derartige Inhaltsstoffe wie sie insbesondere auch für kosmetische Mittel verwendet werden können sind Emulgatoren, Öle, Wachse, Fette oder andere übliche Bestandteile einer kosmetischen Formulierung wie Alkohole, Polyole, Polymere, Schaumstabilisatoren, Elektrolyte, Öle, flüchtige Kohlenwasserstoffe, Silikonderivate oder Silikonderivate, Wirkstoffe, Feuchthaltemittel, Füllstoffe, Farbpigmente, Glanzpigmente, Farbstoffe, UV-Filter, Parfümöle, Antioxidantien, Stabilisatoren, entzündungshemmende Zusätze, durchblutungsfördemde Zusätze, Konservierungsmittel, Bakterizide, desodorierend wirkende Substanzen, Antitranspirantien, lnsektenrepellentien, Vitamine, Proteine, Mittel zum Verhindem von Schäumen, Verdickungsmittel, weichmachende Substanzen, anfeuchtende und/oder feuchthaltende Substanzen, usw.

Ein Verzeichnis von möglichen zugelassenen Inhaltsstoffen für kosmetische Mittel, wie sie prinzipiell auch für die vorliegende Erfindung Verwendung finden können, enthält die Broschüre "Kosmetika-Inhaltsstoffe- Funktionen", die vom Industrieverband Körperpflege- und Waschmittel e.V. und dem Fachverband der chemischen Industrie Österreichs, Berufsgruppe Körperpflegemittel, Frankfurt am Main / Wien, Juni 1998, herausgegeben worden ist.
Medizinische topische Zubereitungen enthalten in der Regel ein oder mehrere Medikamente in wirksamer Konzentration.

Zur Unterscheidung zwischen kosmetischer und medizinischer Anwendung und den entsprechenden Produkten werden auf die geltenden Bestimmungen von Deutschland verwiesen, wie sie z.B. in der Kosmetikverordnung oder dem Lebensmittel- und Arzneimittelgesetz niedergelegt sind.

Es versteht sich die medizinischen Zubereitungen dieselben Inhaltsstoffe wie sie vorstehend beispielhaft für die kosmetische Anwendung genannt worden sind enthalten können, soweit sie für medizinische Zwecke zugelassen sind.

Im allgemeinen werden die jeweiligen Inhaltsstoffe den erfindungsgemäßen Zubereitungen in für den jeweiligen Verwendungszweck üblichen Anteilen zugesetzt.

Die erfindungsgemäß eingesetzten linearen Poly-α-glucane können auch besonders gut als Matrixmaterial für den Zubereitungen zuzusetzende Inhaltsstoffen wirken. Beispielsweise können kosmetische und/oder medizinische Wirkstoffe auf den Poly-α-glucanen adsorbiert und/oder absorbiert vorliegen.

Besondere Eignung als Matrix- oder Trägermaterial haben lineare Poly-α-glucane mit helicaler Tertiärstruktur, wie sie z.B. von dem bevorzugten Poly-1,4-α-D-glucan ausgebildet wird.
In diesen Fall können die Zusatzstoffe wie Wirkstoffe etc. als einschluß in der Helix vorliegen analog z.B. der von den Cyclodextrinen gebildeten Einschlußverbindungen.

Die Herstellung der erfindungsgemäßen Zubereitungen kann nach den üblichen dem Fachmann geläufigen Regeln erfolgen.

Die erfindungsgemäßen Zubereitungen können in beliebiger, für den jeweiligen Anwendungszweck geeigneter Formulierung vorliegen, z.B. als Emulsionen wie O/W-Emulsionen, W/O-Emulsionen, multiple Emulsionen z.B W/OIW-, OIWIO-, OIW/O/W , W/O/W/O- Emulsionen etc., auf Wachsbasis, wasserfrei, Hydrodispersionen, Gele, Öle, wasserfreie Salben bzw. Salbengrundlagen usw.

Die erfindungsgemäß eingesetzten wasserunlöslichen linearen Poly-α-glucane können prinzipiell die Funktionen der üblicherweise verwendeten Polyglucane aus z.B. nativen Quellen wie Stärke in topischen Zubereitungen übemehmen. Gegenüber den Grundstoffen aus nativen Quellen haben sie den Vorteil, daß sie auf einfache Weise mit großer Homogenität und Reinheit erhalten werden können.

So können sie als Füllstoff, Verdickungsmittel, Bindemittel oder Geliermittel eingesetzt werden.

Im Gegensatz zu vielen Pigmenten wie z.B. nicht mikronisiertes Titandioxid, weißein die erfindungsgemäß eingesetzten Poly-α-glucane nicht auf der Haut, d.h. sie wirken transparent und können daher vorteilhaft derartige Pigmente ersetzen.

So können sie als Ersatz für Stoffe wie Talk oder Kaolin genommen werden, die aufgrund ihrer unregelmäßigen oder plättchenförmigen Gestalt zu einem stumpfen Gefühl beim Auftragen führen, z.B. in pigmentierten Produkten, oder als Kompaktierungshilfsmittel in gepressten Pudern eingesetzt werden.

Weiter wurde für die erfindungsgemäß eingesetzten Poly-α-glucane ein absorbierender Effekt beobachtet.
Aufgrund diese absorbierenden Effektes eignen sie sich besonders gut auch als Zusatzstoff in Deodorantien, Körperpuder wie Body Talc, zur Aufnahme von überschüssigen Hautfett z.B. in anti-oil- oder Antiakne-Produkten.

Weiter konnte beobachtet werden, daß sie Hautrauhigkeiten zu reduzieren vermögen, insgesamt einen beruhigenden Effekt auf die Haut haben, sowie eine weichmachende und moisturizierende Wirkung ausüben.
Geeignete Anteile an erfindungsgemäß eingesetzten Poly-α-glucan für Cremes, Lotionen, Make-up, Kompaktcremes und ähnlichem liegen im Bereich von etwa 0,5 Gew.% bis etwa 40 Gew.%, vorzugsweise etwa 2 bis etwa 10 Gew.%, für Puder von etwa 0,5 bis etwa 80 Gew.%, für Salben und pharmazeutische Salben können entsprechende Anteile gewählt werden, wobei die Anteile jeweils auf das Gesamtgewicht der betreffenden Zubereitung bezogen sind.

Es versteht sich, daß bei Bedarf z.B. für spezielle Anwendungen auch mehr z.B. bis zu 100 Gew.%, oder weniger Poly-α-glucan eingesetzt werden kann.

Der Anteil an Poly-α-glucan in den jeweiligen Zubereitungen richtet sich selbstverständlich nach dem erwünschten Effekt und /oder der speziellen Formulierung der Zubereitung.

Nachstehend wird die Erfindung anhand von einzelnen Beispielen veranschaulicht.

Für die Beispiele 1 bis 4 wurde das gemäß Beispiel 5 erhaltene Poly-α-glucan verwendet.

### Beispiele 1 und 2 und Vergleichsbeispiele 1 und 2

### Herstellvorschrift für Lotion und Creme

Die Zusammensetzung für die Lotion (Beispiele 1a, 1b und Vergleichsbeispiel 1) sowie die Creme (Beispiele 2a, 2b und Vergleichsbeispiel 2) sind in Tabelle 1 aufgeführt.

### Vorbereitung:

### Fettphase:

Emulgatoren und Öle unter Rühren auf 70° C erwärmen. Konservierungsmittel hier Parabene zugeben und unter Rühren lösen.

### Wasserphase:

Demin: Wasser auf 70° C erwärmen, restl. Bestandteile unter Rühren lösen. Poty-α-glucan dispergieren.

### Konservierungs-Lösung:

Konservierungsmittel (Imidazolidinyl Urea) in demin. Wasser lösen.

### Herstellung:

Fettphase vorlegen, t = 68-70° C. Wasserphase (t = 68-70° C) unter Rühren und Homogenisieren zugeben. Nach der Zugabe 20 min. rühren und homogenisieren, t = 65-68 C.

### Kühlen:

unter Rühren auf 40° C.
Konservierungsmittel-Lösung und Parfümöl zugeben und 5 min. rühren und homogenisieren.
Unter Rühren auf 30° C kühlen.

Die erfindungsgemäßen Cremes und Lotionen fühlten sich beim Applizieren sehr angenehm an und erzeugten ein samtiges Gefühl auf der Haut.
Der Zusatz von wasserunlöslichen linearen Poly-α-glucan führte zu keinem Weißein auf der Haut.

### Beispiele 3a und b sowie Vergleichsbeispiel 3

### Herstellvorschrift für Compact Cream Make up

Die Zusammensetzung ist in Tabelle 2 wiedergegeben.

### Vorbereitung:

Lanolin und Öle auf 80-82° C erwärmen. Antioxydant und Filmbildner unter Rühren lösen. Vorgeschmolzene Wachse (t = 80° C) zugeben.

### Herstellung:

Vormischung aus Farbpigmenten, Titandioxid, Talkum, Mica und Poly-α-glucan unter Rühren und Homogenisieren zugeben.
Anschl. 30 min. rühren und homogenisieren, t = 82° C.

### Entleeren:

Bulk luftfrei in geeignete Behältnisse entleeren.

Im Vergleich zur Basisrezeptur zeigte die erfindungsgemäße Zubereitung eine reduzierte Wachsartigkeit.

### Beispiele 4a und b und Vergleichsbeispiel 4

### Herstellvorschrift für Foundation

Die Zusammensetzung ist in Tabelle 3 wiedergegeben.

### Vorbereitung:

Emulgator, Konsistenzgeber und Öl auf 70° C erwärmen und mischen. Konservierung zugeben und unter Rühren lösen.

Demin. Wasser und Prophylenglycol auf 70° C erwärmen, Wirkstoff und Stabilisator unter Rühren lösen. Vormischung aus Farbpigmenten, Titandioxid und Poly-α-glucan bei laufendem Homogenisator einsaugen. Anschließend 30 min. rühren und homogenisieren.

### Herstellung:

Filtrierte Fettphase (t = 70° C) unter Rühren und Homogenisieren einsaugen und weitere 10 min. rühren und homogenisieren, t = 65° C.

### Kühlen:

unter Rühren auf 40° C. Vorlösung Konservierung in demin. Wasser und Parfümöl bei 40°C zugeben. Anschließend 10 min. rühren und homogenisieren.
Unter Rühren und Homogenisieren auf 30° C kühlen.

Im Gegensatz zu der Grundierung gemäß Vergleichsbeispiel fühlte sich die erfindungsgemäße Grundierung deutlich cremiger an. Zudem wurde eine Viskositätssteigerung beobachtet.

**Tabelle 1**

| Komponente | Vergleich 1 | 1a | 1b | Vergleich 2 | 2a | 2b |
|---|---|---|---|---|---|---|
| Sorbitan Stearate | 1,50 | 1,50 | 1,50 | 1,50 | 1,50 | 1,50 |
| Polysorbate 60 | 2,00 | 2,00 | 2,00 | 2,00 | 2,00 | 2,00 |
| Cetyl Alcohol | | | | 2,50 | 2,50 | 2,50 |
| Mineral Oil | 8,00 | 8,00 | 8,00 | 8,00 | 8,00 | 8,00 |
| Decyl Oleate | 4,00 | 4,00 | 4,00 | 4,00 | 4,00 | 4,00 |
| Glyceryl Stearate | 4,00 | 4,00 | 4,00 | 5,00 | 5,00 | 5,00 |
| Dimethicone | 0,50 | 0,50 | 0,50 | 0,20 | 0,20 | 0,20 |
| Parabene | 0,35 | 0,35 | 0,35 | 0,35 | 0,35 | 0,35 |
| | | | | | | |
| | | | | | | |
| Vollentsalztes Wasser (Aqua) | 63,30 | 63,30 | 63,30 | 58,60 | 58,60 | 58,60 |
| Allantoin | 0,20 | 0,20 | 0,20 | 0,20 | 0,20 | 0,20 |
| Propylenglycol | 3,50 | 3,50 | 3,50 | 5,00 | 5,00 | 5,00 |
| | | | | | | |
| | | | | | | |
| vollentsalztes Wasser (Aqua) | 10,00 | 8,00 | 5,00 | 10,00 | 8,00 | 5,00 |
| Poly-α-glucan* | | 2,00 | 5,00 | | 2,00 | 5,00 |
| vollentsalztes Wasser (Aqua) | 2,00 | 2,00 | 2,00 | 2,00 | 2,00 | 2,00 |
| lmidazolidinyl Harnstoff | 0,50 | 0,50 | 0,50 | 0,50 | 0,50 | 0,50 |
| | | | | | | |
| | | | | | | |
| Parfüm (Fragrance) | 0,15 | 0,15 | 0,15 | 0,15 | 0,15 | 0,15 |
| | | | | | | |
| | 100,00 | 100,00 | 100,00 | 100,00 | 100,00 | 100,00 |

| | | | | | | |
|---|---|---|---|---|---|---|
| * keine INCI Bezeichnung | | | | | | |

**Tabelle 2**

| Komponente (INCI) | Vergleich 3 | 3a | 3b |
|---|---|---|---|
| Lanolin | 10,10 | 10,10 | 10,10 |
| MineralOil | 7,85 | 7,85 | 7,85 |
| Castor Oil | 2,80 | 2,80 | 2,80 |
| Octydodecanol | 22,00 | 20,00 | 17,00 |
| Isopropyl Palmitate | 5,00 | 5,00 | 5,00 |
| Antioxydant | 0,05 | 0,05 | 0,05 |
| PVP/Eicosane Copolymer | 0,15 | 0,15 | 0,15 |
| Eisenoxid | 0,68 | 0,68 | 0,68 |
| Titandioxid * | 2,75 | 2,75 | 2,75 |
| Talcum * | 16,07 | 16,07 | 16,07 |
| Mica' | 13,90 | 13,90 | 13,90 |
| Pofy-α-glucan* | | 2,00 | 5,00 |
| Cera Microcritallina | 9,70 | 9,70 | 9,70 |
| Petrolatum | 7,65 | 7,65 | 7,65 |
| Camauba | 1,30 | 1,30 | 1,30 |
| | | | |
| | 100,00 | 100,00 | 100,00 |

| | | | |
|---|---|---|---|
| * keine INCI Bezeichnung | | | |

**Tabelle 3**

| Komponente INCI | Vergleich 4 | 4a | 4b |
|---|---|---|---|
| Glyceryl Stearate | 9,00 | 9,00 | 9,00 |
| lsopropyl Myristate | 2,25 | 2,25 | 2,25 |
| Cetyl Alcohol | 1,50 | 1,50 | 1,50 |
| Parabene | 0,35 | 0,35 | 0,35 |
| | | | |
| | | | |
| Vollentsalztes Wasser (Aqua) | 62,27 | 62,27 | 62,27 |
| Allantoin | 0,25 | 0,25 | 0,25 |
| Xanthan Gum | 0,30 | 0,30 | 0,30 |
| | | | |
| | | | |
| Propylenglycol | 8,90 | 8,90 | 8,90 |
| Eisenoxid | 0,68 | 0,68 | 0,68 |
| Titiandioxid * | 2,75 | 2,75 | 2,75 |
| | | | |
| | | | |
| Vollentsalztes Wasser (Aqua) | 10,00 | 8,00 | 5,00 |
| Poly-α-glucan* | | 2,00 | 5,00 |
| | | | |
| | | | |
| Parfüm (Fragance) | 0,25 | 0,25 | 0,25 |
| Vollentsalztes Wasser (Aqua) | 1,00 | 1,00 | 1,00 |
| Imidazolidinyl Urea | 0,50 | 0,50 0,50 | 0,50 |
| | | | |
| | 100,00 | 100,00 | 100,00 |

| | | | |
|---|---|---|---|
| * keine INCI Bezeichnung | | | |

### Beispiel 5

In-vitro-Produktion von Poly-1,4-α-D-glucan in einem biokatalytischen Prozeß mit Hilfe von Amylosucrase.

In einem sterilisierten (Dampfsterilisation) 15 1 Gefäß werden 10 l einer 20%igen Saccharose-Lösung gegeben. Der Enzymextrakt, Amylosucrase enthaltend, wird in einer Portion zugegeben. Die Enzymaktivität beträgt in diesem Experiment 16 units. Die Apparatur wird mit einem ebenfalls sterilisierten KPG-Rührer versehen. Das Gefäß wird verschlossen und bei 37°C aufbewahrt und gerührt. Bereits nach einer Zeit von wenigen Stunden bildet sich ein weißer Niederschlag. Die Reaktion wird nach einer Zeitdauer von 180 Stunden beendet. Der Niederschlag wird abfiltriert und zur Abtrennung niedermolekularer Zucker fünf Mal mit Wasser gewaschen. Der im Filter verbleibende Rückstand wird bei 40°C im Trockenschrank unter Anlegung eines Vakuums mit Hilfe einer Membranpumpe (Firma Vacuubrand GmbH & Co, CVC 2) getrocknet. Die Masse beträgt 685 g (Ausbeute 69 %).

### Beispiel 6

Charakterisierung des mit Amylosucrase synthetisierten wasserunlöslichen 1,4-α-D-linearen Poty-1,4-α,-D-glucans aus Beispiel 1

Es werden 2 mg des Poly-1,4-α-D-glucans aus Beispiel 1 bei Raumtemperatur in Dimethylsulfoxid (DMSO, p.a. von Riedel-de-Haen) gelöst und filtriert (2 µm Filter). Ein Teil der Lösung wird in eine Gelpermeationschromatographie Säule injiziert. Als Elutionsmittel wird DMSO verwendet. Die Signalintensität wird mittels eines RI-Detektors gemessen und gegen Pullutanstandards (Firma Polymer Standard Systems) ausgewertet. Die Flußrate beträgt 1.0 ml pro Minute.

Die Messung ergibt ein Zahlenmittel des Molekulargewichts (Mₙ) von 14.200 g/mol und ein Gewichtsmittel des Molekulargewichts (M_{w}) von 29.500 g/mol. Dies entspricht einer Dispersität von 2,1.

## Patentansprüche

1. Topische Zubereitung, die als wesentlichen Bestandteil mindestens ein biotechnisch erhaltenes, wasserunlösliches lineares Poly-α-1,4,-D-Glucan enthält, welches in 4-Position keine Verzweigungen enthält, und wobei das Poly-α-1,4,-D-Glucan nicht in Form von Sphärischen Mikropartlkeln mit einem mittleren Durchmesser von 1 nm bis 100 Mikrometern vorliegt, und wobei weiter das Poly-α-1,4,-D-Glucan biokatalytisch durch katalytische Reaktion von monomeren Grumdbausteinen, wie oligomeren Sacchanden, bevorzugt von Mono-und/oder Disacchariden unter Verwendung eines Biokatalysators, bevorzugt eines Enzyms, besonders bevorzugt der Amylosucrase, hergestellt ist.

2. Topische Zubereitung nach Anspuch 1 **dadurch gekennzeichnet, daß** das Poly-α-glucan in der topischen Zubereitung in einer Menge von etwa 0,5 bis etwa 80 Gew. % enthalten ist, bezogen auf das Gesamtgewicht der topischen Zubereitung.

3. Topische Zubereitung nach einem der vorhergehenden Ansprüche, **dadurch gekennzeichnet, daß** die topische Zubereitung ein kosmetisches Mittel wie Körperpflegemittel oder eine dekorative Kosmetik ist.

4. Topische Zubereitung nach einem der Ansprüche 1 bis 2, **dadurch gekennzeichnet, daß** die topische Zubereitung eine human- oder tiermedizinische Zubereitung zur äußeren Applikation ist.

5. Topische Zubereitung nach einem der vorhergehenden Ansprüche, **dadurch gekennzeichnet, daß** die topische Zubereitung ausgewählt ist unter Cremes, Kompaktcremes, Lotionen, Milchen, Masken, Puder, Salben, Salbengrundlagen und Seifen.

6. Topische Zubereitung nach Anspruch 5, **dadurch gekennzeichnet, daß** die topische Zubereitung für die dekorative Kosmetik ist und ausgewählt ist unter Cremes, Puder oder Fonds für Make-up.

7. Topische Zubereitung nach Anspruch 6, **dadurch gekennzeichnet, daß** das Make-up Rouge, Lidschatten oder Lippenstift ist.

8. Verwendung von biotechnisch erhaltenen, wasserunlöslichen linearen Poly-α-glucan, wie in Anspruch 1 definiert, in einer topischen .Zubereitung.

## Claims

1. A topical preparation which comprises, as essential constituent, at least one biotechnologically obtained water-insoluble linear poly-α-1,4,-D-glucan having no branches at the 4-position, and at which the poly-α-1,4-D-glucan is not present in the form of spheric micro particles having an average diameter from 1 nm to 100 micrometers, and at which the poly-α-1,4-D-glucan has been bio catalytically produced by catalytic reaction of monomeric basic building blocks, such as oligomer saccharides, preferably mono- and/or di-saccharides by use of a biocatalyst, preferably an enzyme, inparticuler preferred Amylosucrase.

2. The topical preparation as claimed in claim 1,wherein the poly-α-glucan in the topical preparation is present in an amount of from approximately 0,5 to approximately 80 % by weight, based on the total weight of the topical preparation.

3. The topical preparation as claimed in any of the preceding claims, wherein the topical preparation is a cosmetic composition, such as bodycare composition or a decorative cosmetic.

4. The topical preparation as claimed in any of claims 1 to 2, wherein the topical preparation is a human or veterinary medical preparation for external application.

5. The topical preparation as claimed in any of the preceding claims, wherein the topical preparation is chosen from creams, compact creams, lotions, milks, masks, powders, ointments, ointment bases and soaps.

6. The topical preparation as claimed in claim 5, wherein the topical preparation is for decorative cosmetics and is chosen from creams, powders or bases for make-up.

7. The topical preparation as claimed in claim 6, wherein the make-up is blusher, eye shadow or lipstick.

8. The use ofbiotechnologically obtained, water-insoluble linear poly-α-glucan as defined in claim 1 in a topical preparation.

## Revendications

1. Préparation topique qui contient comme composante principale au moins un poly-α -1,4,-D-glucane linéaire, insoluble dans l'eau, conservé de manière biotechnique, qui dans la position 4 ne contient pas des ramifications et où le poly-α-1,4,-D-glucane ne se présente pas sous forme de microparticules sphériques, avec un diamètre moyen de 1 mn jusqu'à 100 micromètres, et où en continuation le poly-α-1,4,-D-glucane est un produit biocatalytique par la réaction catalytique des blocs fonctionnels sur base de monomères comme les saccharides oligomères, de préférence des mono-et/ou disaccharides par l'utilisation d'un biocatalyseur, de préférence d'une enzyme, spécialement d'une amylosucrase.

2. Préparation topique selon la revendication 1, **caractérisée en ce que**, le poly-α glucane est contenu dans la préparation topique en proportion d'environ 0,5 jusqu'à 80 pour cent de la masse, rapporté à la masse totale de la préparation topique.

3. Préparation topique selon l'une des revendications antérieures, **caractérisée en ce que**, la préparation topique est un produit cosmétique, comme serait un produit à soigner le corps ou un produit cosmétique décoratif.

4. Préparation topique selon la revendication 1 ou 2, **caractérisée en ce que**, la préparation topique est une préparation pour la médecine humaine ou vétérinaire, pour application extérieure.

5. Préparation topique selon l'une des revendications antérieures, **caractérisée en ce que**, la préparation topique est sous forme de crèmes, crèmes compactes, lotions, lait, masques, poudres, onguents, éléments se basant sur onguents, et savons.

6. Préparation topique selon la revendication 5, **caractérisée en ce que**, la préparation topique est pour la cosmétique décorative et se présente sous forme de crèmes, poudres ou fonds de make-up.

7. Préparation topique selon la revendication 6, **caractérisée en ce que**, le make-up signifie le rouge de maquillage, fard ou rouge à lèvres.

8. Utilisation dans une préparation topique d'un poly-α-glucane linéaire, insoluble dans l'eau, conservée de manière biotechnique ainsi qu'elle est définie dans la revendication 1.
